# EUROPEAN PATENT SPECIFICATION

(11) **EP 4 056 114 B1**
(45) Date of publication and mention of the grant of the patent: **21.01.2026**
(21) Application number: 21162254.3
(22) Date of filing: 12.03.2021
(51) Int. Cl.: A61B 5/087, A61B 5/00, A61B 5/085, A61B 5/08

(54) **COMPUTER-IMPLEMENTED METHOD FOR EXTRACTING RESPIRATORY INFORMATION FROM A BIO-IMPEDANCE SIGNAL**
COMPUTERIMPLEMENTIERTES VERFAHREN ZUR GEWINNUNG VON ATMUNGSINFORMATIONEN AUS EINEM BIOIMPEDANZSIGNAL
PROCÉDÉ MIS EN OEUVRE PAR ORDINATEUR POUR EXTRAIRE DES INFORMATIONS RESPIRATOIRES D'UN SIGNAL DE BIO-IMPÉDANCE

(43) Date of publication of application: 14.09.2022
(73) Proprietor: Onera Technologies B.V., 5617 BD Eindhoven (NL)
(72) Inventor: Rossi, Alessandro C., 5617 BD Eindhoven (NL); Schneider, Hartmut, 5617 BD Eindhoven (NL)
(74) Representative: AWA Sweden AB

(56) References cited:
- CN-A- 112 022 123
- VILLE-PEKKA SEPPA ET AL: "Assessment of Pulmonary Flow Using Impedance Pneumography", IEEE TRANSACTIONS ON BIOMEDICAL ENGINEERING, IEEE SERVICE CENTER, PISCATAWAY, NJ, USA, vol. 57, no. 9, 1 September 2010 (2010-09-01), pages 2277 - 2285, XP011339357, ISSN: 0018-9294, DOI: 10.1109/TBME.2010.2051668
- LEO PEKKA MALMBERG ET AL: "Measurement of tidal breathing flows in infants using impedance pneumography", EUROPEAN RESPIRATORY JOURNAL, vol. 49, no. 2, 19 December 2016 (2016-12-19), GB, pages 1600926, XP055646104, ISSN: 0903-1936, DOI: 10.1183/13993003.00926-2016
- JAKKAEW PRASARA ET AL: "An Approach to Non-contact Monitoring of Respiratory Rate and Breathing Pattern Based on Slow Motion Images", 2019 IEEE INTERNATIONAL CONFERENCE ON CONSUMER ELECTRONICS - ASIA (ICCE-ASIA), IEEE, 12 June 2019 (2019-06-12), pages 47 - 51, XP033678424, [retrieved on 20191224]
- BROWNE ET AL: "A multiscale polynomial filter for adaptive smoothing", DIGITAL SIGNAL PROCESSING, ACADEMIC PRESS, ORLANDO,FL, US, vol. 17, no. 1, 2 December 2006 (2006-12-02), pages 69 - 75, XP005724174, ISSN: 1051-2004, DOI: 10.1016/J.DSP.2006.01.006

## Description

### Technical Field

The present invention relates to a method for extracting respiratory information from a bio-impedance signal, and in particular a method for extracting respiratory information from a bio-impedance signal by means of computationally efficient filters.

### Background

When assessing the health condition of a subject, it is common to monitor the subject's respiration. One way of doing so relies on extracting respiratory information from a subject's bio-impedance signals, in particular when acquired using electrodes placed on the torso of a subject. This is possible since the bio-impedance signal is dependent on the content and distribution of tissues and materials along the path of an injected current; hence, air flowing in and out of the lungs and bronchus will cause measurable variations in accordance with the respiratory behavior of the subject.

However, the bio-impedance signal also includes disturbances that are mostly related to other tissue components (skin, muscle, fat, blood) and their periodic motion throughout respiratory and cardiac cycles. Therefore, it is challenging to separate respiratory information from disturbances present in the bio-impedance signal, which can often be in partially overlapping frequency bands.

Thus, there is a need for a method for extracting respiratory information from a bio-impedance signal in an accurate and computationally efficient manner.

Known methods using bio-impedance signals are disclosed in e.g. CN112022123A; Ville-Pekka Seppa et al: "Assessment of Pulmonary Flow Using Impedance Pneumography", IEEE Transactions on Biomedical Engineering, vol. 57, no. 9, 1 September 2010, pages 2277-2285; Leo Pekka Malmberg et al: "Measurement of tidal breathing flows in infants using impedance pneumography", European Respiratory Journal, vol. 49, no. 2, 19 December 2016, page 1600926; Jakkaew Prasara et al: "An Approach to Non-contact Monitoring of Respiratory Rate and Breathing Pattern Based on Slow Motion Images", 2019 IEEE International Conference on Consumer Electronics - Asia, 12 June 2019, pages 47-51; and Browne et al: "A multiscale polynominal filter for adaptive smoothing", Digital Signal Processing, Academic Press, Orlando, FL, US, vol. 17, no. 1, 2 December 2016, pages 69-75.

### Summary

It is an object of the present invention to provide a method for extracting accurate and precise respiratory information from a bio-impedance signal in a computationally efficient manner.

The invention is primarily based on the inventor's realization that using a Savitzky-Golay low pass filter incorporating a 3^{rd} degree polynomial is particularly useful for enabling accurate and precise respiratory information to be extracted from bio-impedance signals. The reason for this is that such a Savitzky-Golay filter is able to smooth data sets for increasing precision of the data without distorting it to unacceptable levels. It is therefore useful in applications of extracting qualitative information from relatively noisy signals, such as extracting respiratory information from subject measurement signals, e.g. bio-impedance signals. Further, Savitzky-Golay filters incorporating a 3^{rd} degree polynomial have relatively little computational cost as compared to other smoothing techniques. It is therefore useful in applications mandating rapid computation, such as real-time monitoring of respiratory information of a subject.

Moreover, when differentiating a respiratory effort signal characterized by little noise by means of a Savitzky-Golay derivative kernel incorporating a 2^{nd} degree polynomial fit, it has been identified that additional accurate and precise respiratory information representing a respiratory flow signal can be extracted. Obtaining a respiratory effort signal characterized by little noise is obtained as a consequence of applying a Savitzky-Golay low pass filter incorporating a 3^{rd} degree polynomial fit on a bio-impedance signal. This filtering cascade allows our method to track relative breath-to-breath amplitude changes in both respiratory effort and respiratory flow and comparing such effort and flow relative changes to each other in order to assess whether upper airway obstructions are present. The aspects of the invention can be summarized by the appended claims.

### Brief Description of the Drawings

The invention will in the following be described in more detail with reference to the enclosed drawings, wherein:
Fig. 1 shows a chart of the method according to the first and second aspect of the invention respectively - dashed segments indicate a further embodiment(s);
Fig. 2 shows diagrams of a provided bio-impedance signal and the resulting respiratory effort signal and respiratory flow signal obtained by the present invention according to one embodiment of the invention;
Fig. 3 shows a block diagram of the components of a device according to one embodiment of the invention wherein dashed segments indicate a further embodiment of the invention.

### Description of Embodiments

The present invention will be described more fully hereinafter with reference to the accompanying drawings, in which preferred embodiments of the invention are shown. This invention may, however, be embodied in many different forms and should not be construed as limited to the embodiments set forth herein; rather, these embodiments are provided so that this disclosure will be thorough and complete, and will fully convey the scope of the invention to those skilled in the art. In the drawings, like numbers refer to like elements.

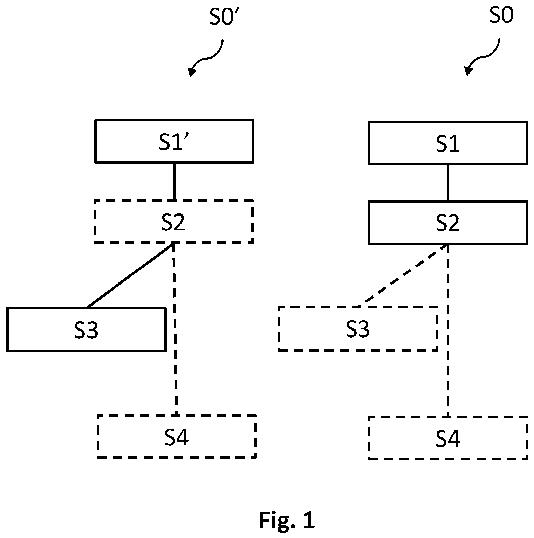

Fig. 1 shows a chart of the method according to the first and second aspect of the invention respectively - dashed segments indicate a further embodiment(s). The methods differ in that one method S0 relates to providing a respiratory effort signal while the other method S0' relates to providing a respiratory flow signal.

The method S0 according to one embodiment comprises the step of providing S1 a bio-impedance signal of a subject. The bio-impedance signal is measured by means of electrodes arranged on the torso of the subject, preferably in a tetrapolar configuration. The method further comprises a step of filtering S2 the bio-impedance signal using a Savitzky-Golay low-pass filter to provide first respiratory information representing a respiratory effort signal. The Savitzky-Golay low-pass filter incorporates a 3^{rd} order polynomial fit. Further, the Savtizky-Golay low-pass filter is using a frame length between 1 - 2 seconds.

By this, it is possible to extract respiratory information which is both accurate and precise. Further, it is a very computationally efficient method to extract such respiratory information and may advantageously be used in applications mandating rapid computation, such as real-time monitoring of respiratory information.

According to one further embodiment, which is indicated in Fig. 1, the method S0 comprises a step of differentiating S3 the respiratory effort signal with a Savitzky-Golay first derivative kernel to provide second respiratory information representing a respiratory flow signal. The Savtizky-Golay first derivative kernel incorporates a 2^{nd} order polynomial fit. The Savitzky-Golay first derivative kernel is applied on subsets of datapoints of the respiratory effort signal which are determined using a frame length between 0.5 - 1.5 seconds. Further, the Savtizky-Golay first derivative kernel is applied on the respiratory effort signal, optionally without it first having been normalized. The resulting respiratory flow signal may then be normalized.

The method S0' according to one embodiment comprises the step of providing S1' a noise filtered respiratory effort signal. Preferably, the noise filtered respiratory effort signal is a respiratory effort signal extracted from a bio-impedance signal, which respiratory effort signal then is noise filtered if so required. The method further comprises a step of differentiating S3 the noise filtered respiratory effort signal with a Savitzky-Golay first derivative kernel to provide second respiratory information representing a respiratory flow signa. The Savtizky-Golay first derivative kernel incorporates a 2^{nd} degree polynomial fit.

According to one further embodiment, the respiratory effort signal is extracted from the bio-impedance signal by filtering S2 the bio-impedance signal using a Savitzky-Golay low-pass filter incorporating a 3^{rd} degree polynomial fit.

Either of the methods S0, S0' comprises in further embodiments a step S4 of filtering the respiratory effort signal using a high-pass filter to obtain a baseline-filtered bio-impedance signal. The high-pass filter used is a high-pass zero-phase Butterworth filter of 5^{th} order with a low cut-off frequency in the range of 0.001 - 0.1 Hz. Other high-pass filter may be viable depending on parameters.

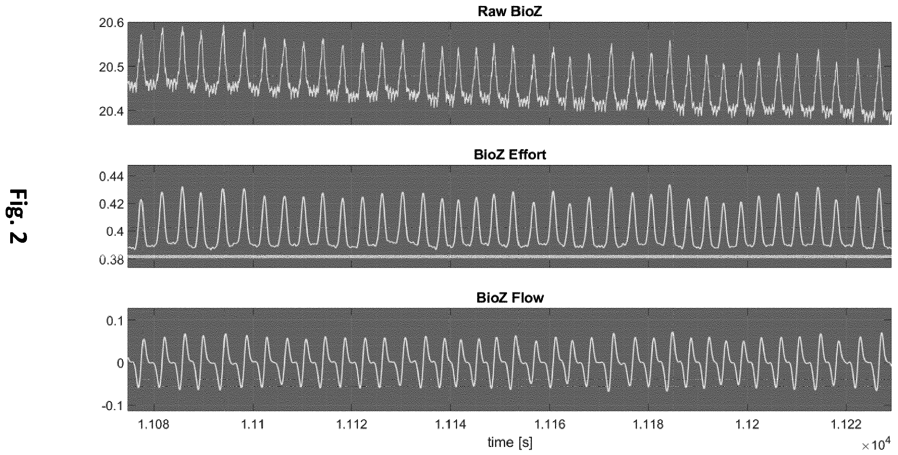

Fig. 2 shows diagrams of a provided bio-impedance signal and the resulting respiratory effort signal and respiratory flow signal obtained by the present invention according to one embodiment of the invention. The top graph shows a provided bio-impedance signal. The middle graph shows the resulting respiratory effort signal after being normalized in the [0, 1] range. This particular respiratory effort shown is a segment from a signal which was achieved with a low-pass Savtizky-Golay filter incorporating a 3^{rd} order polynomial fit and a frame-length of 1.55 seconds, and a high-pass zero-phase Butterworth filter of the 2^{nd} degree with a cut-off frequency of 0.02 Hz and. The bottom graph shows the resulting respiratory flow signal after being normalized in the [-1, 1] range. This particular flow signal was achieved by filtering the respiratory effort signal in the middle graph (shown in arbitrary units, but still having a non-zero baseline) using a Savitzky-Golay kernel incorporating a 2^{nd} degree polynomial fit with a frame length of 1 second.

The choice of a 2^{nd} degree 1^{st}-derivative Savitzky-Golay filter cascaded to the aforementioned 3^{rd} degree low-pass Savitzky-Golay filter enables the derivation of a smooth respiratory flow signal from the bio-impedance signal, which still retains fast variations possibly associated to clinically relevant breathing events.

According to one aspect of the invention, a computer program is provided also. The computer program comprises instructions which, when executed by a computing device, cause the computing device to carry out the method according to the first or second aspect of the invention, or any embodiment thereof. The computer program may be stored on a program readable storage medium.

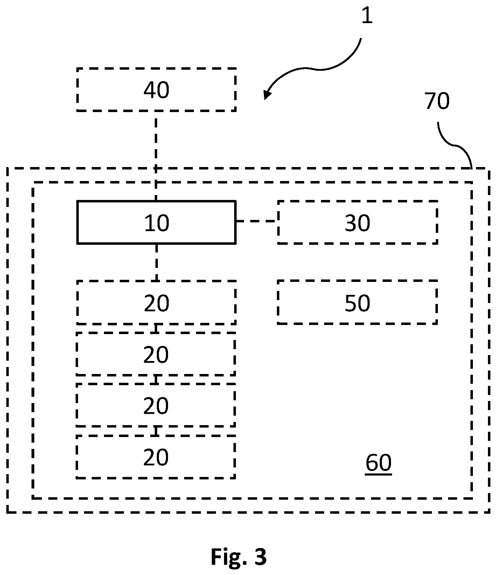

Fig. 3 shows a block diagram of the components of a device according to one embodiment of the invention wherein dashed segments indicate a further embodiment of the invention.

The device 1 is configured for extracting respiratory information from a bio-impedance signal. The device 1 comprises a processor 10 configured to execute the method S0, S0' according to the first or second aspect of the invention, or any embodiments thereof. The device 1 may comprise other electronics components such as one or more program readable storage mediums 20, a communications module 30, a power source 50 and a circuit board 60. The device 1 may comprise a housing 70 configured to house the processor 10 and other electronics components. The device 1 may also comprise one or more electrodes 40 configured to be arranged on the subject for measuring a bio-impedance, for example in tetra-polar configuration on the subject's torso.

The device 1 may be a wearable patch device.

The one or more program readable storage mediums 20 may be configured to store extracted respiratory information and/or Savtizky-Golay filter parameters, and/or in the case the Savitzky-Golay filter or filters are configured to be adaptively updated, updated Savitkzy-Golay filter parameters.

The communications module 30 may be configured to enable the device 1 to receive and process filter control data, which control data specify control data of the Savitzky-Glay filter or filters.

In the drawings and specification, there have been disclosed preferred embodiments and examples of the invention and, although specific terms are employed, they are used in a generic and descriptive sense only and not for the purpose of limitation, the scope of the invention being set forth in the following claims.

## Claims

1. A computer-implemented method (S0) for extracting respiratory information from a bio-impedance signal, the method comprising the steps of:
providing (S1) a bio-impedance signal of a subject;
filtering (S2) the bio-impedance signal using a Savitzky-Golay low-pass filter to provide first respiratory information representing a respiratory effort signal;
wherein the Savitzky-Golay low-pass filter incorporates a 3^{rd} degree polynomial fit,
wherein the Savitzky-Golay low-pass filter is applied on subsets of data points of the bio-impedance signal which are filtered using a frame length in the interval of 1.3 - 1.7 seconds;
differentiating (S3) the respiratory effort signal with a Savitzky-Golay first derivative kernel to provide second respiratory information representing a respiratory flow signal,
wherein the Savitzky-Golay first derivative kernel incorporates a 2^{nd} degree polynomial fit;
wherein the Savitzky-Golay first derivative kernel is applied on subsets of datapoints of the respiratory effort signal which are filtered using a frame length in the interval of 0.5-1.5 seconds, preferably between 0.8-1.2 seconds.

2. Method (S0, S0') according to claim 1, comprising a step of filtering (S4) the respiratory effort signal using a high-pass filter to obtain a baseline filtered respiratory effort signal.

3. Method (S0, S0') according to any of claims 1-2, wherein the Savitzky-Golay filter or filters are adaptively updated based on an instantaneous respiratory frequency.

4. Method (S0, S0') according to claim 3, wherein the instantaneous respiratory frequency is estimated from the bio-impedance signal.

5. Method (S0, S0') according to claim 2, wherein the high-pass filter has a cutoff frequency between 0.001 - 0.1 Hz, preferably between 0.01 - 0.05 Hz.

6. A computer program comprising instructions which, when executed by a computing device, cause the computing device to carry out the method according to any of claims 1 - 5.

7. A computer readable storage medium storing the computer program according to claim 6.

## Patentansprüche

1. Computerimplementiertes Verfahren (S0) zur Gewinnung von Atmungsinformationen aus einem Bioimpedanzsignal, wobei das Verfahren die folgenden Schritte umfasst:
Bereitstellen (S1) eines Bioimpedanzsignals eines Patienten;
Filtern (S2) des Bioimpedanzsignals unter Verwendung eines Savitzky-Golay-Tiefpassfilters, um erste Atmungsinformationen bereitzustellen, die ein Atemanstrengungssignal darstellen;
wobei das Savitzky-Golay-Tiefpassfilter eine Polynomanpassung 3. Grades enthält,
wobei das Savitzky-Golay-Tiefpassfilter auf Teilmengen von Datenpunkten des Bioimpedanzsignals angewandt wird, die unter Verwendung einer Rahmenlänge im Intervall zwischen 1,3 und 1,7 Sekunden gefiltert werden;
Differenzieren (S3) des Atemanstrengungssignals mit einem ersten Savitzky-Golay-Ableitungskern, um zweite Atmungsinformationen bereitzustellen, die ein Atemflusssignal darstellen,
wobei der erste Savitzky-Golay-Ableitungskern eine Polynomanpassung 2. Grades enthält;
wobei der erste Savitzky-Golay-Ableitungskern auf Teilmengen von Datenpunkten des Atemanstrengungssignals angewandt wird, die unter Verwendung einer Rahmenlänge im Intervall zwischen 0,5 und 1,5 Sekunden, vorzugsweise zwischen 0,8 und 1,2 Sekunden, gefiltert werden.

2. Verfahren (S0, S0') nach Anspruch 1, umfassend einen Schritt des Filterns (S4) des Atemanstrengungssignals unter Verwendung eines Hochpassfilters, um ein basisliniengefiltertes Atemanstrengungssignal zu erhalten.

3. Verfahren (S0, S0') nach einem der Ansprüche 1 bis 2, wobei das oder die Savitzky-Golay-Filter adaptiv auf der Grundlage einer momentanen Atemfrequenz aktualisiert wird bzw. werden.

4. Verfahren (S0, S0') nach Anspruch 3, wobei die momentane Atemfrequenz von dem Bioimpedanzsignal geschätzt wird.

5. Verfahren (S0, S0') nach Anspruch 2, wobei das Hochpassfilter eine Grenzfrequenz zwischen 0,001 und 0,1 Hz, vorzugsweise zwischen 0,01 und 0,05 Hz, aufweist.

6. Computerprogramm, das Anweisungen umfasst, die, wenn sie von einer Rechenvorrichtung ausgeführt werden, die Rechenvorrichtung veranlassen, das Verfahren nach einem der Ansprüche 1 bis 5 durchzuführen.

7. Computerlesbarer Datenträger, der das Computerprogramm nach Anspruch 6 speichert.

## Revendications

1. Procédé mis en œuvre par ordinateur (S0) pour extraire des informations respiratoires d'un signal de bio-impédance, le procédé comprenant les étapes suivantes :
la fourniture (S1) d'un signal de bio-impédance d'un sujet ;
le filtrage (S2) du signal de bio-impédance à l'aide d'un filtre passe-bas Savitzky-Golay pour fournir des premières informations respiratoires représentant un signal d'effort respiratoire ;
dans lequel le filtre passe-bas Savitzky-Golay incorpore un ajustement polynomial de 3^{ème} degré,
dans lequel le filtre passe-bas Savitzky-Golay est appliqué à des sous-ensembles de points de données du signal de bio-impédance qui sont filtrés à l'aide d'une longueur de trame dans l'intervalle de 1,3 à 1,7 seconde ;
la différenciation (S3) du signal d'effort respiratoire avec un premier noyau de dérivée Savitzky-Golay pour fournir des deuxièmes informations respiratoires représentant un signal de flux respiratoire,
dans lequel le premier noyau de dérivée Savitzky-Golay incorpore un ajustement polynomial de 2^{ème} degré ;
dans lequel le premier noyau de dérivée Savitzky-Golay est appliqué à des sous-ensembles de points de données du signal d'effort respiratoire qui sont filtrés à l'aide d'une longueur de trame dans l'intervalle de 0,5 à 1,5 seconde, de préférence de 0,8 à 1,2 seconde.

2. Procédé (S0, S0') selon la revendication 1, comprenant une étape de filtrage (S4) du signal d'effort respiratoire à l'aide d'un filtre passe-haut pour obtenir un signal d'effort respiratoire filtré de référence.

3. Procédé (S0, S0') selon l'une quelconque des revendications 1 et 2, dans lequel le ou les filtres Savitzky-Golay sont mis à jour de manière adaptative sur la base d'une fréquence respiratoire instantanée.

4. Procédé (S0, S0') selon la revendication 3, dans lequel la fréquence respiratoire instantanée est estimée à partir du signal de bio-impédance.

5. Procédé (S0, S0') selon la revendication 2, dans lequel le filtre passe-haut présente une fréquence de coupure entre 0,001 et 0,1 Hz, de préférence entre 0,01 et 0,05 Hz.

6. Programme informatique comprenant des instructions qui, lorsqu'elles sont exécutées par un dispositif informatique, amènent le dispositif informatique à réaliser le procédé selon l'une quelconque des revendications 1 à 5.

7. Support de stockage lisible par ordinateur stockant le programme informatique selon la revendication 6.
